# EUROPEAN PATENT SPECIFICATION

(11) **EP 3 930 682 B1**
(45) Date of publication and mention of the grant of the patent: **24.12.2025**
(21) Application number: 20713963.5
(22) Date of filing: 26.02.2020
(51) Int. Cl.: A61K 9/00, A61K 31/047, A61P 1/10, B65D 1/04, B65D 30/22, B65D 75/00, A61J 3/00, B65D 21/02, B65D 25/54, B65D 1/02, B65D 75/58

(54) **COMPOSITION AND CONTAINER, KIT, METHOD OF USE**
ZUSAMMENSETZUNG UND BEHÄLTER, KIT, VERFAHREN ZUR VERWENDUNG
COMPOSITION ET RÉCIPIENT, KIT, PROCÉDÉ D'UTILISATION

(30) Priority: 27.02.2019 IT 201900002859
(43) Date of publication of application: 05.01.2022
(73) Proprietor: NTC S.r.l., 20124 Milano (IT)
(72) Inventor: MARCELLONI, Luciano, 20124 Milano (IT); BERTOCCHI, Federico, 20124 Milano (IT)
(74) Representative: Benedetto, Marco
(86) International application number: PCT/IB2020/051655
(87) International publication number: WO 2020/174425

(56) References cited:
- EP-A1- 3 015 102
- WO-A1-2010/141751
- WO-A1-2020/174423
- TAKEUCHI YOSHIKO ET AL: "Characterization of mannitol granules and powder: A comparative study using two flowability testers", INTERNATIONAL JOURNAL OF PHARMACEUTICS, vol. 547, no. 1-2, 24 May 2018 (2018-05-24), NL, pages 106 - 113, XP93076901, ISSN: 0378-5173, DOI: 10.1016/j.ijpharm.2018.05.061
- ANONYMOUS: "Mannitol poeder (100 gram) | PoederWinkel.nl | Caffeine, Inositol en Mannitol poeder bestellen", 1 January 2018 (2018-01-01), XP055641365, Retrieved from the Internet <URL:http://www.poederwinkel.nl/product/mannitol-poeder-100-gr/> [retrieved on 20191112]
- GUSTAVO ANDRADE DE PAULO ET AL: "SAFETY OF MANNITOL USE IN BOWEL PREPARATION: a prospective assessment of intestinal methane (CH4) levels during colonoscopy after mannitol and sodium phosphate (NaP) bowel cleansing", ARQUIVOS DE GASTROENTEROLOGIA, vol. 53, no. 3, 1 September 2016 (2016-09-01), pages 196 - 202, XP055640475, DOI: 10.1590/S0004-28032016000300014
- ROWE RAYMOND C ET AL: "Handbook of pharmaceutical excipients, Mannitol", 1 January 2006, HANDBOOK OF PHARMACEUTICAL EXCIPIENTS, PHARMACEUTICAL PRESS [U.A.], LONDON [U.A.], PAGE(S) 449 - 453, ISBN: 978-1-58212-058-4, XP002537758
- MALORI A: "Oral drug reconstitution: Making it easy and accurate via packaging innovation", vol. 88, 9 July 2018 (2018-07-09), pages 18 - 21, XP009517120, ISSN: 2049-145X, Retrieved from the Internet <URL:https://www.ondrugdelivery.com/magazines/novel-oral-delivery-systems-88/>
- SAHEBALLY S M ET AL: "A randomized controlled trial comparing polyethylene glycol + ascorbic acid with sodium picosulphate + magnesium citrate solution for bowel cleansing prior to colonoscopy", IRISH JOURNAL OF MEDICAL SCIENCE, CAHILL, DUBLIN, IE, vol. 184, no. 4, 26 August 2014 (2014-08-26), pages 819 - 823, XP035605388, ISSN: 0021-1265, [retrieved on 20140826], DOI: 10.1007/S11845-014-1182-4

## Description

The present invention regards a single-dose composition, a container or a heat-sealed sachet comprising such single-dose composition, a kit comprising said single-dose composition, and a method of use of a kit comprising the aforementioned single-dose composition.

Mannitol (CAS N° 69-65-8; otherwise referred to as "D-mannitol" or "mannite") is a chiral alditol, with six hydroxyl groups on an aliphatic chain consisting of six saturated carbon atoms.

At room temperature, mannitol appears as a white, odourless and non-hygroscopic solid. Mannitol is used as a sweetener in foods for diabetic people due to the fact that it is poorly absorbed by the bowel.

Over the years, mannitol has been widely used in the pharmaceutical industry and in the gastroenterological field due to its osmotic diuretic characteristics and by virtue of the fact that it is an ancient remedy for constipation. The sweet taste of mannite makes its use particularly appreciated by children.

Mannose, an oxidation product of mannitol, has also proven to be particularly suitable for use in paediatrics, since it acts as a prebiotic for bacterial strains of the gut flora.

A use of mannitol that has been investigated in the past is its use in emptying the colon prior to endoscopic examinations, carried out by ingesting high doses of mannitol pre-dissolved in water. In particular, the action of emptying the colon occurs in 4 or 5 hours after ingesting mannitol pre-dissolved in water. The use of mannitol pre-dissolved in water has a number of advantages over other known substances having the function of emptying the colon (for example Macrogol^{®}, based on polyethylene glycol). As a matter of fact, Macrogol^{®}, having a slower induction, requires at least one first intake the day before, and a second intake on the day of the examination. Furthermore, the taste of some of such known substances is markedly worse, being very bitter, with respect to the taste of mannitol.

Nevertheless, the use of mannitol solutions reveals some limitations and drawbacks. As a matter of fact, as mentioned in GA de Paulo et al. (Arq Gastroenterol. 53(3), 2016, pages 196-202), ir the past there have been cases of bowel explosion in patients who had been subjected to gastric emptying by ingesting mannitol pre-dissolved in water. These explosions have been attributed to incomplete cleansing of the colon, with permanence - in the bowel - of methane-producing bacteria, or hydrogen-reducing producers (see for example the technical problem of the prior art document EP3015102A1, paragraphs [0002]-[0012]). Specifically, following some cases, it was observed that a portion of mannitol that remained undissolved in the solution taken by the patient had reduced the ingested dose that would have been required for proper bowel cleansing and, as discussed above, only a partial dissolution of mannitol in solution can be a contributing cause of explosion.

According to a further aspect, in cases where the user of the preparation is an elderly person, this could limit the shaking required for the full dissolution of mannitol in solution due to various factors, by way of example, due to pain in the joints, distraction, lack of patience, or the like.

Moreover, an incomplete intake of mannitol in solution, and therefore an incomplete cleansing of the bowel could also limit the possibilities of a correct diagnosis, which may not be useful in a statistically larger number of patients.

It is therefore extremely important that, for this specific use, mannitol be completely dissolved in solution, so that it can be administered effectively, completely and reproducibly.

Thus, the present invention falls within the preceding context, aiming at providing a single-dose composition, a preferably single-dose container, a kit for the use of a kit comprising a single-dose composition and a method capable of: (1) obtaining a quantitative dissolution of mannitol, uniformly reproducible in substantially all cases of administration prior to the performance of colonoscopy; (2) reducing up to eliminating the presence of the mannitol precipitate in the solution prior to administration thereof to a patient, and, thus, (3) allowing a simple, safe and effective administration of mannitol to any patient under normal conditions of use of the latter. Moreover, the manufacture, handling and packing of the present powder composition, in an extremely fine granulometric state, represents a major technological challenge.

Such objectives are achieved by means of a single-dose composition according to claim 1, by means of said composition for use in a treatment as defined in claim 3, by means of a preferably single-dose container or of a sachet according to claims 4, 5, or 12, by means of a kit comprising a single-dose composition according to claim 7, and by means of a method of use of a kit according to claim 10. The claims dependent on the aforementioned claims show preferred embodiments of the present invention.

The present invention will now be described based on the attached drawings, provided solely by way of non-limiting example, wherein:
- figure 1 shows a container subject of the present invention, according to a first embodiment, wherein the sealing element is spaced from the container compartment;
- figure 2 shows a schematic view of a kit subject of the present invention, according to a possible embodiment, wherein the container is shaped differently with respect to figure 1;

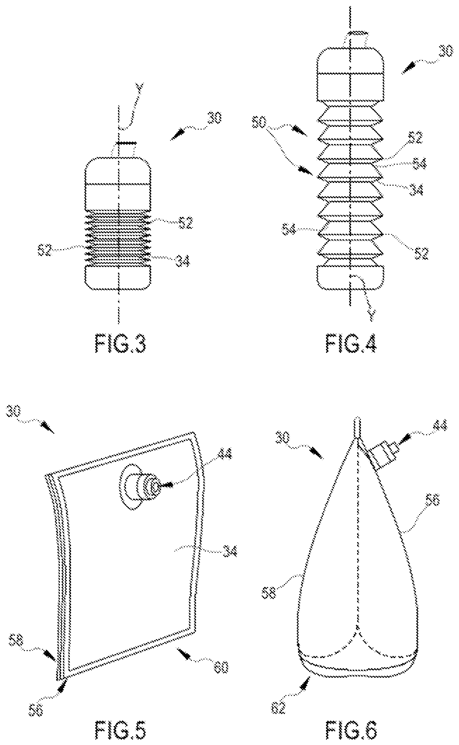

- figures 3, 4 show a dissolving container according to a first embodiment, respectively in an at least partially collapsed configuration and in an expanded configuration;
- figures 5, 6 show a dissolving container according to a second embodiment, respectively in an at least partially (for example: completely) collapsed configuration and in an expanded configuration;
- figures 7, 8 show a container and dissolving container assembly according to another embodiment, respectively in a lateral view and in a cross section at the plane VIII-VIII schematised in figure 7;
- figure 9 shows a plan view of a heat-sealed sachet 1 subject of the present invention, according to a possible embodiment.

It should be observed that the dimensional ratios shown in the previous figures do not represent limitations to the present invention.

A single-dose composition according to the present invention comprises or consists of mannitol at an amount comprised from 50 to 200 grams, wherein such mannitol is in powder form. This powder has a bulk density comprised from 0.50 to 0.62 g/ml and it comprises powder particles. A percentage comprised from 90% to 100% by weight of the powder particles has particle size distribution comprised from 1 µm to 500 µm, preferably from 5 µm to 400 µm, more preferably from 10 µm to 300 µm.

It should be observed that the bulk density values mentioned in this description are to be understood as being measured at ambient temperature and pressure.

As regards the terminology used in this description, the term "single-dose" is used to indicate an amount of a single dose of composition, to be used in a single use as described in greater detail hereinafter.

In this regard, it should be pointed out that the weight of mannitol in the present composition is an unusually high amount (comprised from 50 to 200 g, assuming substantially pure mannitol) for a single dose of composition in the pharmaceutical field, which has posed the inventors of the present invention to face new technological challenges. By way of example, the use of a large amount of mannitol has resulted in the unusual use and adaptation of multi-dose containers (i.e. that can be opened and closed several times) to a single use, for single-dose products.

According to an embodiment, the single-dose composition of the present invention consists of an amount comprised from 50 g to 60 g of mannitol According to another embodiment, the single-dose composition of the present invention consists of an amount comprised from 80 g to 120 g, preferably from 85 g to 115 g, more preferably from 90 g to 110 g, even more preferably comprised from 95 g to 105 g of mannitol.

According to a further embodiment, the single-dose composition of the present invention consists of an amount comprised from 110 g to 190 g, preferably comprised from 120 g to 180 g, more preferably comprised from 130 g to 179 g, even more preferably comprised from 135 g to 185 g. Preferably, the single-dose composition of the present invention consists of an amount comprised from 140 g to 170 g, preferably comprised from 141 g to 160 g, more preferably comprised from 145 g to 155 g of mannitol.

In this description, the expression "consisting of" is used to indicate a composition in which mannitol is the essentially exclusive component, except for any impurities present therein.

According to an embodiment, the single-dose composition is devoid of pyrogenic substances and/or devoid of excipients.

More specifically, avoiding the use of excipients which favour densification and/or the flow of mannitol into the container, but which could have simultaneously slowed down the dissolution thereof, was considered a necessity and an attention toward the patient who, following ingestion of the mannitol composition previously dissolved in water, is subjected to an almost full emptying of the bowel in order not to risk administering any unnecessary and foreign component which can in any way interfere with the practices aimed at the pre-treatment and relaxation of the colon walls in order to perform a correct colonoscopy.

According to another embodiment, mannitol has a percentage by weight comprised from 97% to 100%, preferably comprised from 98% to 100% or comprised from 99% to 100%, or of 100%, with respect to the total weight of such composition. By way of example, possible components other than mannitol (when present, at an amount less than 3% by weight with respect to the total weight of the composition) could comprise substances related to mannitol, such as other polyalcohols, for example sorbitol.

According to a preferred embodiment, mannitol has a percentage by weight of 99,5%, of 99,6%, of 99,7%, of 99,8%, of 99,9% or of 100% with respect to the total weight of such composition. Preferably, the mannitol contained in the composition is of a pharmaceutical grade.

It should be observed that, in each range of values mentioned in this description, the extremes of the range are to be deemed within the range (unless the context clearly indicates otherwise), same case applying to any intermediate value between the extremes, although not explicitly stated numerically.

As regards the terms "in powder form", this expression is used to indicate mannitol in finely divided, pulverulent form, comprising a plurality of powder particles.

With regard to the expression "particle size distribution" (PSD), this term is used indicate the statistical dimensional distribution curve of the powder particles.

According to an embodiment, the powder particle size distribution could be as in Table 1 below:

**Table 1**

| **Range** | **% particles** |
|---|---|
| < 100 µm | 25.87% |
| 180 - 100 µm | 54.88% |
| 250 - 180 µm | 16.59% |
| 365 - 250 µm | 0,96% |
| 500 - 365 µm | 1,27% |
| 600 - 500 µm | 0,43% |
| ≥ 600 µm | 0,59% |

According to an embodiment, the powder particles could be substantially spherical-shaped.

This means that the particle size distribution discussed in this description could be a "particle size diameter" with the same described characteristics, preferably comprised from 1 µm to 500 µm, preferably comprised from 5 µm to 400 µm, more preferably comprised from 10 µm to 300 µm, even more preferably comprised from 15 µm to 250 µm, without prejudice to possible variability (±) comprised from 1% to 15% (as discussed below).

As concerns the embodiments that provide for "substantially spherical" powder particles, it should be observed that the sphericity parameter is defined by the ratio between the outer surface of a powder particle and the outer surface of an equivalent sphere (i.e. a sphere of the same volume as the powder particle).

In such context, a powder particle is deemed to be "substantially spherical" if it has a sphericity parameter comprised from 1 to 1,3, preferably comprised from 1 to 1,2, more preferably comprised from 1 to 1,15, even more preferably comprised from 1 to 1,1 or comprised from 1 to 1,05.

As regards the bulk density outlined above, in particular the poured bulk density, such parameter is measured according to the European Pharmacopoeia reference standard (Ph. Eur.), current edition 2.2.42, in force at the priority date of this patent application.

In the context of the present invention, the expression "bulk" implies that the powder density value is calculated in a manner formally analogous to an absolute density (such as for a solid body or for a liquid). However, since a powder has empty inter-particle spaces, the total volume occupied by the powder (i.e. its outer bulk), thus including the inter-particle spaces between the various particles, should be taken into account when evaluating bulk density.

More precisely, according to the aforementioned regulation, a 100 ml class A graduated flask is filled and brought to volume with the powder particles, after which the bulk density is calculated as the ratio between the weight of the powder contained in the flask and the volume (100 ml) occupied by that weight of the powder particles.

The bulk density of the mannitol powder inside the container is comprised from 0,50 g/ml to 0,62 g/ml, preferably from 0,56 g/ml to 0,60 g/ml, even more preferably comprised from 0,57 g/ml to 0,59 g/ml.

According to another embodiment, the bulk density of mannitol powder is 0,58 g/ml.

According to an embodiment, a method that can be used for analysing the particle size distribution is discussed hereinafter: 100 g of mannitol powder are screened using 600 µm, 500 µm, 355 µm, 250 µm, 180 µm and 100 µm ASTM series sieves. All sieves except for the 100 µm sieve come from GIULIANI TECNOLOGIE S.r.l. (Via Centallo 62/18, 10156 Turin, Italy; https://www.giuliani.it/setacci-sieves). The 100 µm sieve comes from Retsch GmbH (Retsch-Allee 1-5, 42781 Haan, Germany; https://www.retsch.com/products/sieving/). Such sieves were connected to a vibrating screen made by RMU-Resistenze Meccaniche Unificate serial n° 42280, at an intensity of 10, for 13 minutes. Following screening, a particle size profile of the mannitol powder is constructed. Regarding this, see for example the particle size profiles of Table 3 or of Table 4.

As concerns the powder particle size distribution, it should be observed that the particle size distribution will be comprised from 1 µm to 500 µm, unless there is a variability (±) comprised from 1% to 15%, preferably comprised from 1% to 10%, more preferably comprised from 1% to 5%, with respect to the upper limit of the range of 500 µm (or 400 µm, 300 µm, 250 µm according to other embodiments).

According to a particularly preferred embodiment, the powder particles have a particle size distribution comprised from 1 µm to 250 µm, without prejudice to a possible variability (±) comprised from 1% to 15%, preferably comprised from 1% to 10%, more preferably comprised from 1% to 5%, with respect to the upper limit of the range (250 µm). Only those compositions wherein 90 to 100% by weight of the powder particles has a particle size dimension comprised from 1 µm to 500 µm are according to the claimed invention.

According to an advantageous embodiment, the aforementioned single-dose composition, according to any one of the illustrated embodiments, is a single-dose composition for use in the treatment of constipation, or for use as a purgative to be administered to a patient prior to a colonoscopy examination.

The aforementioned objectives are also achieved through a container 1, preferably a single-dose container 1, containing a single-dose composition - according to any one of the preceding claims - in a container compartment 4 hermetically sealed by a removable sealing element 6.

The aforementioned objectives are also achieved through a heat-sealed sachet, preferably a single-dose heat-sealed sachet, containing a single-dose composition - according to any one of the preceding claims - in a container compartment 4 hermetically sealed by a removable or tearable sealing element, wherein said heat-sealed sachet is made of a polymeric material which can also possibly be compostable according to the UNI EN 13432 or ASTM D6400 standard.

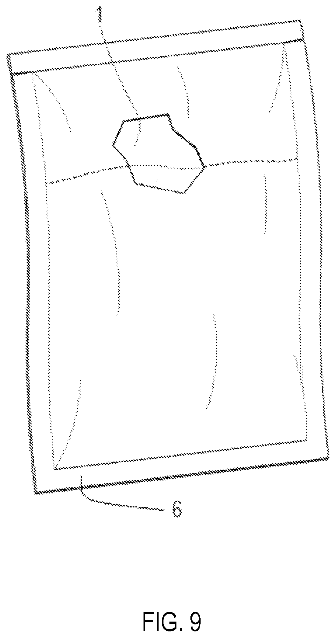

In other words, such container 1 or the heat-sealed sachet (for example shown in figure 9) advantageously forms the primary casing for mannitol in powder form, with which the powder particles are directly at contact.

It should be observed that the expression "hermetically" is used to indicate the ability to at least prevent the powder from leaking out from the container compartment and/or from the inner compartment discussed hereinafter. Preferably, the expression "hermetically" in the context of the dissolving container 30 entails a substantially hermetic sealing of the inner compartment 26 of such dissolving container 30, in order to obtain the single-dose solution.

As regards the standard UNI EN 13432 or ASTM D6400 mentioned above, such standard is meant in the version valid at the priority date of the present patent application.

Preferably, the compostable polymeric material comprises or, alternatively, consists of at least one polylactic acid (PLA) film.

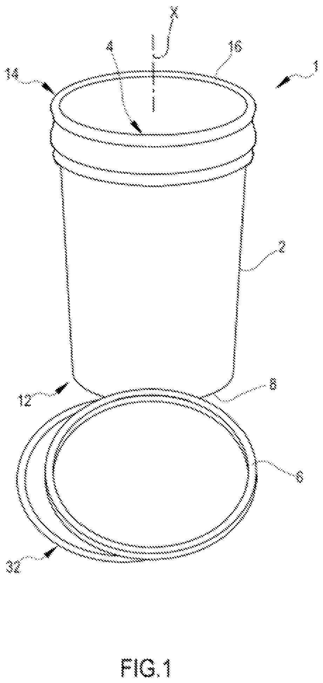

With reference for example to the embodiment of figure 1, the container 1 comprises a first lateral wall 2 which extends around a first main extension axis X so as to delimit the container compartment 4.

According to an embodiment, the first lateral wall 2 is connected to a first bottom wall 8 at a first axial end 12 of the container 1 and, at an opposite second axial end 14, it delimits at least one opening 16 for access to the container compartment 4.

According to another embodiment, the container 1 or the first lateral wall 2 could be substantially cylindrical-shaped or truncated-cone-shaped.

According to a further embodiment, the container 1, or the first lateral wall 2 and the optional first bottom wall 8, could be made of a polymeric material, preferably polyethylene (preferably high-density polyethylene; HDPE) or polypropylene.

According to an embodiment, the sealing element 6 can be connected to the first lateral wall 2, in particular at the second axial end 14 of the container 1, so as to hermetically seal the container compartment 4.

According to another embodiment, the sealing element 6 is made of a more flexible material with respect to the material that the container or the first lateral wall 2 is made of, said material being preferably polymeric such as low-density polyethylene (LDPE).

According to a further embodiment, the sealing element 6 and the container 1 (or the first lateral wall 2 of the latter) could be joined by a tamper-proof seal 32, preferably annular-shaped and/or in the form of a flange.

Therefore, according to such embodiment, before accessing the single-dose composition it will be necessary to tear and/or remove the tamper-proof seal 32, and to remove the sealing element 6.

According to an embodiment, the tamper-proof seal 32 could be designed so as to act as a stirrer, following the tearing or removal thereof. More precisely, the torn or removed seal could be elongated-shaped, so as to be inserted into the container compartment 4 and/or into the inner compartment 26 and be actuated manually in order to facilitate the dissolution of the mannitol powder.

Preferably, the polymeric material of the container 1 (or, of the first lateral wall 2 and of the optional first bottom wall 8) is non-identical/different with respect to the polymeric material that the sealing element 6 is made of.

Preferably, the polymeric material of the sealing element 6 could be polyethylene (preferably low-density polyethylene; LDPE).

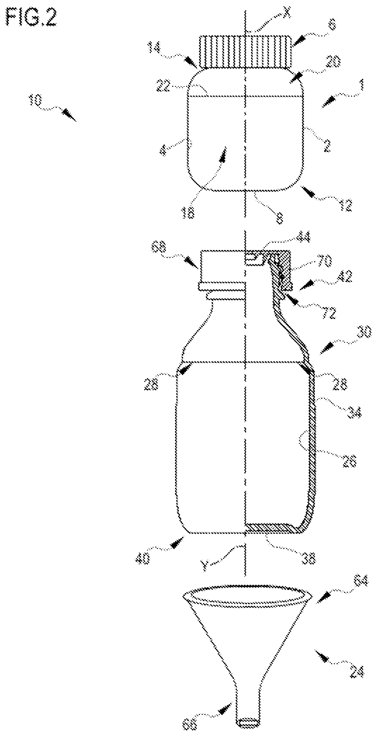

According to an embodiment, the heat-sealed sachet, preferably a heat-sealed closed sachet, preferably having 4 ends (4 sides, perimeter ends) heat-sealed for example with a square or rectangular shape. Said heat-sealed sachet (Fig. 9; 1) could comprise or, alternatively, consist of a pair of sachet walls made of said polymeric material or of another specific compostable polymer in the form of material film, said sachet walls being heat-sealed to each other along the perimeter by means of one or more heat-sealing areas (Fig. 9; 2). The embodiment of figure 9 shows the sachet wall facing upwards (the opposite wall being concealed from the view), polygonal-shaped, preferably square- or rectangular-shaped. The heat-sealed outer part 6 in Fig. 9 also represents a sealing element which can be removed for example using cutting means such as a pair of shears.

According to an embodiment, the container compartment 4 comprises a first volume fraction 18 and a second volume fraction 20. The first volume fraction 18 is occupied by the single-dose composition, and the second volume fraction 20 is devoid of the aforementioned composition. More precisely, the second volume fraction 20 could contain a gas, preferably air or at least one inert gas.

In other words, above a vacant surface 22 of the single-dose composition 1 in the container compartment 4 there is a space devoid of mannitol corresponding to the second volume fraction 20.

According to another embodiment, the first volume fraction is equal to or greater than about half of a total internal volume of the container compartment 4.

According to a further embodiment, the first volume fraction is about two-thirds of the total internal volume of the container compartment 4, the second volume fraction being consequently one-third of the total internal volume of such compartment 4.

The aforementioned objectives are further achieved by means of a kit comprising the container 1 or the heat-sealed sachet according to any one of the preceding embodiments, a dissolving container 30 at least partially permeable to light, and a funnel 24 for transferring the single-dose composition from the container 1 or from the heat-sealed sachet to the dissolving container 30.

It should be observed that, the expression "at least partially permeable to light", is used to indicate a dissolving container 30 with at least one portion 36 at least partly transparent (for example: a completely transparent container, or a container with at least one transparent portion 36), so as to allow to see - from the outside - at least one part of an inner compartment 26 of such container, in particular without the need to open the latter. This expression will also include a container comprising at least one pair of transparent portions 36, for example diametrically opposite or contralateral.

The at least partial permeability to light has a double utility in the context of the present invention: on the one hand, seeing the inner compartment 26 allows to establish the complete dissolution of mannitol. On the other hand, in some embodiments, such characteristic allows the introduction of a water volume appropriate for dissolution, or in any case, to establish that the water volume already present in the container is sufficient for a quantitative dissolution of mannitol.

The expression "appropriate water volume" therefore refers to the volume of water present or that can be introduced into the inner compartment, appropriate for fully dissolving the mannitol in powder form. According to a first embodiment, a water volume suitable to completely dissolve the single-dose composition contained in the container 1 or in the heat-sealed sachet is contained in the dissolving container 30.

According to a second embodiment, the dissolving container 30 comprises a level gauge 28 for a water volume that can be introduced (i.e. to be introduced) into the dissolving container 30 and suitable to fully dissolve the single-dose composition contained in the container 1 or in the heat-sealed sachet.

In other words, in these two last embodiments the dissolving container 30 could already be filled with the appropriate water volume, or such container 30 could initially be empty but provided with a level gauge 28 so that a user can, on his own account, introduce the appropriate water volume, specifically without making mistakes.

It should be observed that the term "water" has no particular limitations in the present description. According to an embodiment, the water could comprise or consist of deionised water, demineralised water, mineral water (preferably non-carbonated), or mains water.

As a matter of fact, it was observed that any salts previously dissolved in water have no negative effects on the solvation of mannitol, which is surprising since the salts already dissolved in water should compete with mannitol due to the water solvation capacity, and thus they are expected to increase the time required for the dissolution of the mannitol powder.

On the other hand, with the mannitol powder according to the present invention dissolution was not observed to slow down, although the dissolution kinetics justifying the absence of slowing remain yet to be clarified.

According to an embodiment, the water required for the dissolution of mannitol is at a substantially neutral pH (pH 7,0 ± 0,2), or it is weakly basic, preferably at pH 8,0 ± 0,5.

With regard to the level gauge 28, in an embodiment such indicator could comprise or consist of a sign, a notch, a line or an alphanumeric character arranged on the dissolving container 30, in particular at a window or portion 36 at least partially permeable to light.

More precisely, a second lateral wall 34 of the dissolving container could delimit such window 36. According to another embodiment, the second lateral wall 34 of the dissolving container could delimit at least two windows 36, for example contralateral or diametrically opposite, specifically so as to allow a backlit verification of the complete dissolution of mannitol.

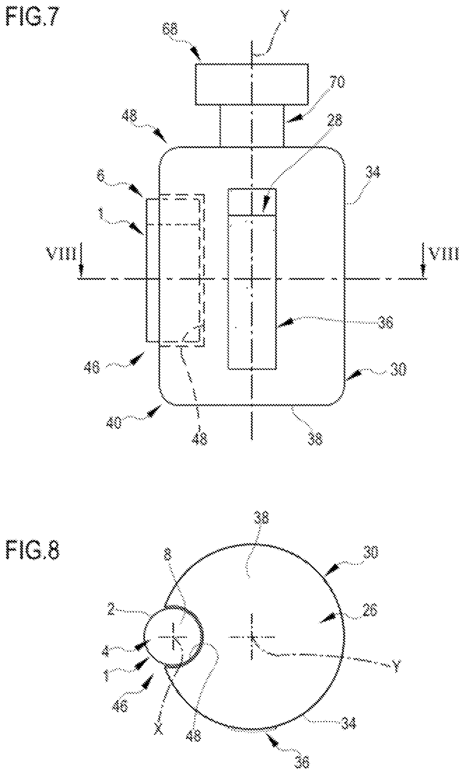

With reference for example to the embodiment of figure 8, the dissolving container 30 comprises the second lateral wall 34 which extends around a second main extension axis Y so as to delimit the inner compartment 26.

According to an embodiment, the second lateral wall 34 is connected to a second bottom wall 38 at a first axial end 40 of the container 30 and, at an opposite second axial end 42, it delimits at least one opening 44 for access to the inner compartment 26.

As regards the characteristics and materials that can be used for the dissolving container 30, the preferred or accessory characteristics mentioned in relation to the container 1 shall apply *mutatis mutandis.*

According to an embodiment, the ratio between the weight of mannitol contained in the container 1 or of the heat-sealed sachet and the water volume contained or that can be introduced up to the level gauge 28 in the dissolving container 30 are mutually selected so as to obtain - at a temperature of 25°C and at ambient pressure - a concentration of mannitol in aqueous solution comprised from 0,05 g/ml to 0,213 g/ml, preferably comprised from 0,1 g/ml to 0,19 g/ml.

According to an embodiment, the container 1 or the heat-sealed sachet is shape-coupled to, or is at least partially nested in, the dissolving container 30.

According to another embodiment, the dissolving container 30 could delimit a coupling seat with the, or a housing seat 46 of the container 1 or with the heat-sealed sachet.

By way of example, the coupling seat or the housing seat 46 could comprise a recess 48 at least partially housing the container 1 or the heat-sealed sachet, for example partially, mainly or substantially fully.

With reference for example to the variant of figures 7 and 8, the second lateral wall 34 is shaped so as to identify the recess 48, being for example designed with a concavity toward the external of the dissolving container 30 (i.e. on the opposite side with respect to the inner compartment 26).

According to an embodiment, when the container 1 is associated with the coupling seat or with the housing seat 46, the main extension axes X, Y are substantially parallel to each other and advantageously non-coincident.

According to another embodiment, at least one second lateral wall 34 of the dissolving container 30 is flexible so that an inner compartment 26 of such container 30 is expandable from an at least partially collapsed configuration to an expanded configuration.

According to a first example, the second lateral wall 34 could comprise at least one bellows-like portion 50, so as to allow conversion between the aforementioned configurations.

More precisely, the second lateral wall 34 could comprise an axial alternation (with respect to the second main extension direction Y) of outer tubular or annular portions 52 arranged side by side and connected to inner tubular or annular portions 54 which form one or more bellows-like portions 50.

In this manner, in the at least partially collapsed configuration, the inner tubular or annular portions 54 are housed (specifically: radially) at least partly in the outer tubular or annular portions 52, so that the volume of the inner compartment 26 is smaller.

However, when the inner compartment 26 is converted into the expanded configuration (for example by pulling on the axial ends 40, 42 of the container), the inner tubular or annular portions 54 move axially alongside the outer tubular or annular portions 52, so that the volume of the inner compartment 26 is greater (at least with respect to the at least partially collapsed configuration).

According to a second example, the second lateral wall 34 could be made of at least one pair of material films 56, 58 joined together (for example sealed), preferably at the respective peripheral portions 60, so as to allow conversion between the aforementioned configurations.

According to such variant, the inner compartment 26 is advantageously enclosed at least by such pair of material films 56, 58.

Advantageously, the two or more material films 56, 58 are joined to each other, so as to form a self-supporting bottom 60 (for example a substantially flat bottom) in the expanded configuration.

As regards to the funnel 24, it comprises a powder-loading portion 64 and a powder-discharge portion 66, the through-flow section through such funnel being tapered at the powder-loading portion 64 toward the powder-discharge portion 66.

According to an embodiment, the powder-loading portion 64 could be truncated-cone or truncated-pyramidal-shaped.

According to another embodiment, the powder-discharge portion 66 could be substantially tubular, or truncated-cone or truncated-pyramidal-shaped.

In a possible embodiment, the kit according to any one of the preceding embodiments is arranged in a secondary, preferably protective, housing.

Lastly, the aforementioned objectives are achieved by means of a method of use of the kit - according to any one of the embodiments illustrated above - comprising the following steps:
i) removing the sealing element from the container 1 or from the heat-sealed sachet (for example, tearing a portion of the heat-sealed sachet);
ii) transferring the single-dose composition from the container 1 or from the heat-sealed sachet to the dissolving container 30 using the funnel 24;
iii) completely dissolving the mannitol powder in the water volume thus obtaining a single-dose solution.

According to an embodiment, the transfer step ii) could comprises the following sub-steps:
ii.a) first pouring at least one part of the appropriate water volume into the container 1 or into the heat-sealed sachet, so as to pre-dissolve the mannitol powder;
ii.b) subsequently transferring the mannitol pre-dissolution of sub-step ii.a) into the dissolving container 30. For example, sub-step ii. a) could comprise at least one (pre-)shaking and/or stirring operation. The term "(pre-)shaking" is used to indicate a pre-shaking and/or shaking operation.

As a matter of fact, mannitol is a poorly flowing powder, hence this embodiment allows to avoid annoying clogging of the funnel due to the increased flowability conferred by water.

According to an embodiment, the container 1 or the heat-sealed sachet could comprise a further level gauge. In this manner, during sub-step ii. a), a part of the water volume could be poured into the container compartment 4 until it corresponds with such further level gauge.

In quantitative terms, according to an embodiment, the single-dose solution of step iii) could contain about 50 g of mannitol in at least 350 ml of water (preferably about 50 g of mannitol in 500 ml of water).

According to another embodiment, the single-dose solution of step iii) could contain about 100 g of mannitol in at least 600 ml of water (preferably about 100 g of mannitol in 700 ml of water).

According to a further embodiment, the single-dose solution of step iii) could contain about 150 g of mannitol in at least 800 ml of water (preferably about 150 g of mannitol in 900 ml of water).

In any case, the principles which should be observed in the determination of the amount of mannitol powder and the appropriate water volume are: a) the desired efficacy for the single-dose solution; and b) not exceeding the saturation concentration at the solution consumption temperature.

Preferably, the dissolving step iii) could comprise the following sub-steps:
iii.a) hermetically sealing (for example closing) the dissolving container 30;
iii.b) shaking the dissolving container 30 of sub-step iii.a) until, using a portion 36 of said container at least partially permeable to light, the precipitates / sediments in the single-dose solution disappear.

With regard to sub-step iii. a), the dissolving container could comprise a cap or closure member 68, which can be reversibly connected to the dissolving container 30, for example to a container neck 70 of the latter.

By way of example, the cap or closure member 68 and the dissolving container 30 (or container neck 70) could be provided with complementary threaded means 72.

As regards the execution times of step iii. b), in an embodiment the shaking could be carried out - for a mass of single-dose composition comprised from 50 to 100 g - for a time comprised from 15 seconds to 2 minutes.

Some examples will be provided hereinafter solely by way of non-limiting example.

### EXAMPLES.

### Example 1: characterisation of mannitol.

The specifications of a mannitol that can be used according to the present invention are shown in Table 2 below. The tests were conducted according to the European Pharmacopoeia reference standards in force at the priority date of the present application.

The tested product is a pharmaceutical grade mannitol named Pearlitol^{®} PF, cod. 050054, manufactured by Faravelli Group S.p.A.

**Table 2**

| **Parameters** | **Specifications** |
|---|---|
| Identity (IR) | Test passed |
| D-Mannitol assay (HPLC) | 97,0 - 102,0% |
| Appearance of the solution | Clear colourless |
| Melting point | 165 - 170°C |
| Reducing sugars | 0,1% max |
| Impurity A: D-Sorbitol | 2,0% max |
| Sum of other impurities (B and C) | 2,0% max |
| Impurities not specified | 0,10% max |
| Total impurities | 2,0% max |
| Heavy metals | ≤ 5ppm |
| Nickel | 1 ppm max. |
| Loss on drying (at 105°C, up to constant weight) | ≤ 0,50% |
| Total aerobic microbial count | 100 CFU/g max |
| Total yeast and mould count | 100 CFU/g max |
| *Escherichia coli* | absent in 10g |
| *Salmonella* | absent in 10g |

### Example 2: Particle size profile.

The particle size profile of three different samples, distinguished by the batch number in Table 3 below, was verified before and after sieving carried out for uniforming the in-line filling of the containers at the industrial level.

**Table 3**

| **Sieve** | **Range** | **Batch 1803711** | | **Batch 1803848** | | **Batch 1804020** | |
|---|---|---|---|---|---|---|---|
| | | **before** | **after** | **before** | **after** | **before** | **after** |
| Precipitate | < 100 µm | 28.75% | 33.54% | 26.96% | 27.58% | 29.40% | 28.35% |
| 100 µm | 180 - 100 µm | 51.46% | 49.68% | 52.38% | 55.88% | 55.08% | 52.04% |
| 180 µm | 250 - 180 µm | 9.97% | 13.09% | 16.55% | 14.91% | 12.75% | 15.54% |
| 250 µm | 365 - 250 µm | 1.00% | 1.57% | 1.52% | 1.06% | 0.98% | 0.88% |
| 365 µm | 500 - 365 µm | 0.85% | 1.46% | 0.98% | 1.15% | 0.85% | 0.92% |
| 500 µm | 600 - 500 µm | 0.25% | 0.66% | 0.38% | 0.42% | 0.23% | 0.26% |
| 600 µm | ≥ 600 µm | 7.72% | 3.16% | 1.24% | 1.11% | 0.85% | 0.11% |

From the previous table it can be observed that the sieving has a break-up effect with respect to the coarser grain size fractions (≥ 600 µm), whose fragmentation enriches by various percentages the finer grain size fractions 100-250 µm.

The grain size of the single-dose composition in a further productive batch is summarised in Table 4 below, whereas the last column on the right shows the percentage by weight of each of the fractions identified in the central column:

**Table 4**

| **Sieve** | **Range** | **Batch P01/18** |
|---|---|---|
| Precipitate | < 100 µm | 25,87% |
| 100 µm | 180 - 100 µm | 54,88% |
| 180 µm | 250 - 180 µm | 16,59% |
| 250 µm | 365 - 250 µm | 0,96% |
| 365 µm | 500 - 365 µm | 1,27% |
| 500 µm | 600 - 500 µm | 0,43% |
| 600 µm | ≥ 600 µm | 0,59% |

### Example 3: Dissolution study as a function of the pH value of water.

The solubility of the mannitol powder was then studied in the following three types of non-carbonated commercial waters:
- Sangemini: pH at source 6,2;
- Antica Fonte Boario: pH at source 7,0;
- Panna Toscana: pH at source 8,0.

The results obtained are shown in Table 5 below:

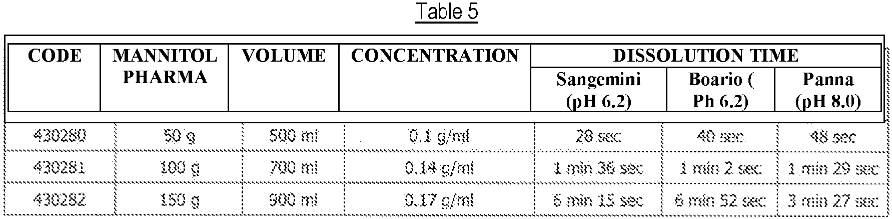

A difficulty of dissolving 150 g of mannitol in 900 ml of water can be deduced from the table above: complete dissolution takes more than 6 minutes for water with weakly acidic or neutral pH, but this time is substantially half as much under weakly basic pH conditions.

Such circumstance is odd and still unexplained in the case of the most critical amount to be dissolved, 150 g: the experimental evidence shows that a slightly basic pH accelerates the dissolution times, even if it has no theoretical assumption since mannitol (itself slightly basic in solution), is expected to dissolve better at slightly acidic pH.

However, an acceleration of dissolution as a function of pH values cannot be observed for samples with 100 g or 50 g of mannitol.

### Example 4: Procedure for filling single-dose containers.

Mannitol powder is a poorly flowing powder, which has a certain tendency, just like all fine powders, to agglomerate under pressure. A drum centrifugal sieve shaker, through which all the mass of the powder particles is passed before the mixing prior to the metering in the container, is used to break up any large powder lumps.

A mesh with a 3mm clear span, much larger than the size or particle size diameter of mannitol (preferably at least 90% with a diameter comprised from 1 µm to 250 µm) was used.

Usually this step further reduces the density of the powders which quickly impact against the edges of the openings of the mesh, causing a decrease in density due to an increase in the disorderly arrangement of the particles which - based on previous evidence - can increase the volume of the material even by 30%.

It has now surprisingly been observed that the use of such sieve shaker allows to accelerate the process for the in-line filling of the single-dose containers up to 50%, reducing the downtime and the onset of waste due to defective filling.

Given the size of the mannitol particles, the passing through a mesh much larger than previously indicated would not be effective in reducing the size thereof, but it shows to be extremely useful in orienting and packing the powder particles, allowing to obtain - in a short through-passing - a much greater transient density, of about 0,73 g/ml, measured in the laboratory, which considerably facilitates a faster descent of the powder into the disposable container.

This behaviour seems to stem from the orientation of the particles that pack better together. Furthermore, with a material already densified at the time of metering in the disposable container, this allows to reduce the metering times and to use smaller bottles, without further settling in the volume that would inevitably occur as the filled disposable container advances toward the labelling.

### Example 5: Dissolution study as a function of the particle size distribution and the density of the mannitol powder used.

The dissolution rate of two types of powder mannitol was compared:
- Type 1: a mannitol subject of the present invention (Pearlitol^{®} PF; according to Example 1) in powder form with a bulk density comprised from 0,40 to 0,65 g/ml and the particle size profile is that of Table 6 below:

**Table 6**

| **sieve (µm)** | **tare (g)** | **gross (g)** | **net (g)** | **%** | **RANGE** |
|---|---|---|---|---|---|
| **600** | 441.41 | 442.95 | 1.54 | 1.539538139 | > 600 |
| **500** | 438.42 | 438.65 | 0.23 | 0.229931021 | 600 - 500 |
| **365** | 395.27 | 396.64 | 1.37 | 1.36958123 | 500 - 355 |
| **250** | 404.29 | 405.19 | 0.9 | 0.899730081 | 355 - 250 |
| **180** | 399.1 | 407.24 | 8.14 | 8.137558732 | 250 - 180 |
| **Precipitate (<180)** | 389.34 | 477.13 | 87.79 | 87.7636709 | < 100 |
| *total* | | | *98.43* | *98.40047986* | |

Therefore, in Type 1 mannitol about 96.6% by weight of the powder particles has a particle size distribution comprised from 1 µm to 500 µm;
- Type 2: a reference mannitol (Pearlitol^{®} 500 DC, marketed by Roquette) in powder form with a bulk density of about 0,673 / 0,683 g/ml (values obtained in two different measurements) and wherein 100% by weight of the powder particles has a particle size distribution greater than 500 µm, in particular comprised from 520 µm to 850 µm. This size of the powder particles was obtained through subsequent sieving operations.

From the operational point of view, the dissolution tests were conducted following the reference analytical specifications of the finished product: "Solve *the prescribed quantity of product powder in the indicated quantity of purified water. Manually stir with* a *stick until the dissolution is complete. Measure the time needed to complete solubilization."* The distilled water used had a temperature of about 19,4°C and the tests were conducted at atmospheric pressure (1 atm), and the stirring is of the manual type carried out by an expert analyst. The results of the present dissolution study are reported in Table 7 below.

**Table 7**

| Type | Particle size distribution | Weight of dissolved powder (in gr) | Water volume (in ml) | Dissolution time | |
|---|---|---|---|---|---|
| | | | | min | sec |
| 1 | < 500 µm | 50 | 500 | 0 | 44 |
| 1 | < 500 µm | 150 | 1000 | 0 | 51 |
| 2 | > 500 µm | 50 | 500 | 3 | 44 |
| 2 | > 500 µm | 150 | 1000 | 7 | 40 |

Table 7 above shows that - in both conducted tests - the mannitol in powder form subject of the present invention has a dissolution rate below one minute. Considering the same conditions, Type 2 powder mannitol (with higher density and particle size distribution) takes more than 3 minutes and more than 7 minutes to dissolve.

Example 6: Stability data of mannitol in powder form subject of the present invention.

Stability of mannitol in the form of powder subject of the present invention was tested and received in a heat-sealed sachet made of compostable material. 50 grams of said powder were stored for 6 months at 40°C/75% RH (Test 1) and for 6 months at 25°C/60% RH (Test 2). Tests were conducted to verify the parameters indicated in Tables 8 and 9 below at the times T0-T6 identified as: T0 = moment of insertion of the air-conditioned environment, T1 = after one month from time T0, T2 = after two months from time T0, T3 = after three months from time T0, T4 = after four months from time T0, T5 = after five months from time T0, T6 = after six months from time T0.

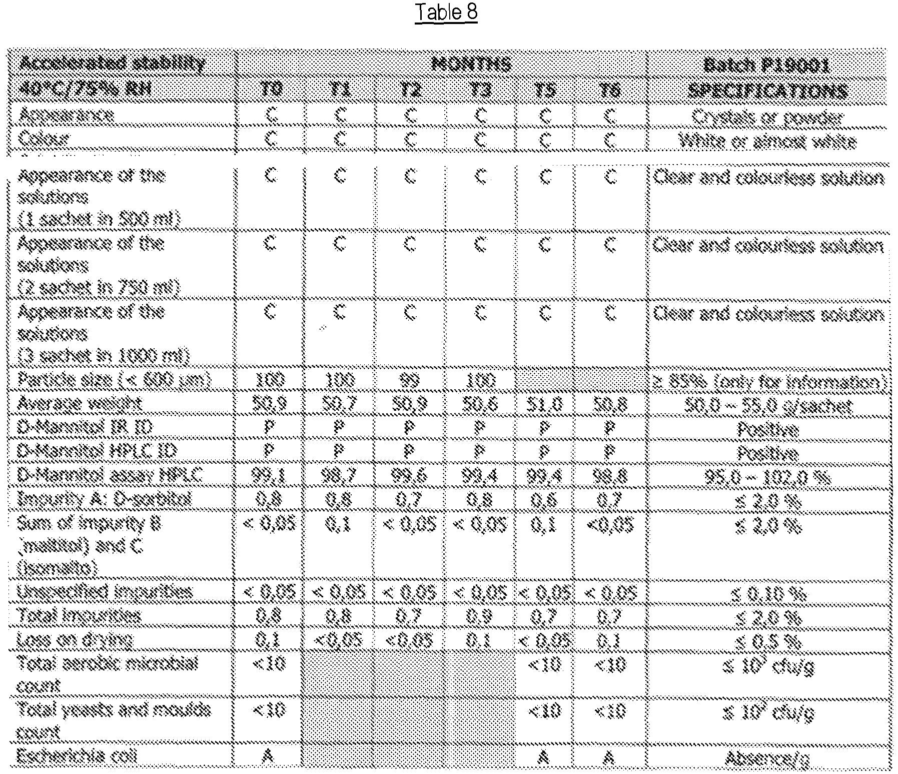

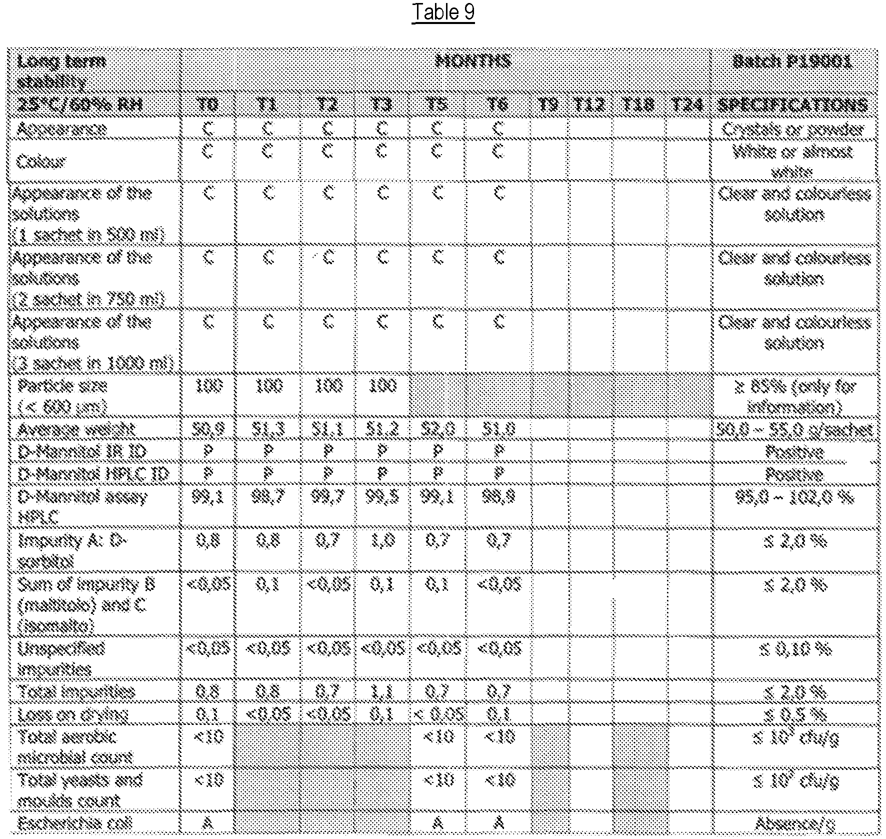

Table 8 and Table 9 above confirm that, under conditions of accelerated stability and long-term stability in the climatic area II, the measured parameters of the mannitol powder contained in the heat-sealed sachet subject of the present invention remained stable throughout the duration of the test.

Innovatively, the present invention allowed to achieve the pre-set objectives.

More precisely, the present invention allows to obtain a complete dissolution of the mannitol powder easily and under the working conditions of a final user.

Advantageously, the characteristics of the sealing element and of the container were specially designed so as to ensure good storage as well as an easy opening of the container by a user.

Advantageously, the pH values explained above allow to accelerate the dissolution rate of mannitol, and thus to make the use of the present composition easier.

Advantageously, the design of the dissolving container allows to save a large amount of space, and/or weight, hence leading to a significant logistic and productive advantage.

Advantageously, the method subject of the present invention allows to compensate for the poor flowability of the mannitol powder, whose transfer is assisted by the solvent.

Advantageously, the heat-sealed sachet containing the mannitol subject of the present invention does not reveal any difficulty of disposal as a special waste, since mannitol is a natural product that does not require particular recovery attention, and the heat-sealed sachet can be disposed of in normal municipal organic waste.

Object of the present invention is
a container (1), preferably a single-dose container or a single-dose heat-sealed sachet, containing a single-dose composition of mannitol in powder form, wherein the single-dose composition of mannitol in powder form is as defined in claim 1;
said container (1) being obtainable or obtained by means of a method comprising the following steps:
   a) breaking-up a coherent mass of powder mannitol, so as to obtain a disintegrated mass from said coherent mass;
   b) filling a plurality of containers with the broken-up mass of step a);
wherein step a) comprises a breaking-up with a centrifugal force, and wherein a bulk density of the coherent mass is smaller than the bulk density of the broken-up mass.

Preferably, said bulk density of the coherent mass is smaller than a bulk density of the broken-up mass by a percentage comprised from 1% to 40%, preferably ranging from 1% to 30%, even more preferably comprised from 5% to 15%, with respect to the bulk density of the broken up mass.

Preferably, said centrifugal force is exerted by means of a drum centrifugal sieve shaker.

Preferably, step a) comprises the following sub-steps:
a.i) sieving the coherent mass of mannitol powder; and
a.ii) packing the product of sub-step a.i).

Preferably, in sub-step a.ii) - the mannitol powder is forced through a mesh with a clear span comprised from 2.0 to 5.0 millimetres, preferably comprised from 2,0 to 4.0 millimetres, more preferably comprised from 2.5 to 3.5 millimetres, even more preferably of 3.0 millimetres.

Preferably, said bulk density of the mannitol powder at the end of sub-step a.ii) is comprised from 0.66 to 0.90 g/ml, preferably comprised from 0.66 to 0.84 g/ml, more preferably comprised from 0.68 to 0.78 g/ml, even more preferably comprised from 0.70 to 0.75 g/ml.

At the end of step b), the mannitol powder in the container has a bulk density comprised from 0.50 to 0.62 g/ml.

At the end of step b), the mannitol powder comprises an amount comprised from 90% to 100% by weight of powder particles with a particle size distribution comprised from 1 µm to 500 µm, preferably comprised from 1 µm to 400 µm, more preferably comprised from 1 µm to 300 µm.

Preferably, the single-dose composition is devoid of excipients and/or pyrogenic substances.

Preferably, said container (1) delimits a container compartment (4), wherein the container compartment (4) comprises a first volume fraction and a second volume fraction, and wherein - at the end of step b) - the first volume fraction is occupied by the single-dose composition, and the second volume fraction of said compartment is devoid of said composition, and preferably wherein the first volume fraction is about two-thirds of a total internal volume of the container compartment (4), the second volume fraction being about one-third of the total internal volume.

Said single-dose composition comprises, or consists, of mannitol at an amount comprised from 50 to 200 grams, wherein said mannitol is in form of powder, wherein said powder has a bulk density comprised from 0. 50 to 0.62 g/ml and it comprises powder particles, and wherein a percentage comprised from 90% to 100% by weight of the powder particles has a particle size distribution comprised from 1 µm to 500 µm.

Preferably, the mannitol contained in said single-dose composition is present at a percentage by weight comprised from 97% to 100%, preferably a percentage by weight of 100%, with respect to the total weight of said composition, wherein said bulk density of said mannitol powder is comprised from 0.50 g/ml to 0.62 g/ml, and wherein the single-dose composition is devoid of excipients and/or pyrogenic substances.

Preferably, the single-dose composition contained therein is for use in the treatment of constipation, or for use as a purgative to be administered to a patient prior to a colonoscopy examination.

Preferably, the container (1), preferably a single-dose container or a single-dose heat-sealed sachet, wherein said single-dose composition according as defined above, is contained in a container compartment (4) hermetically sealed by a removable sealing element (6).

Preferably, said container is a heat-sealed sachet, preferably a single-dose heat-sealed sachet, containing a single-dose composition as defined above, in a container compartment (4) hermetically sealed by a removable or tearable sealing element, wherein said heat-sealed sachet is made of a polymeric material compostable according to the UNI EN 13432 or ASTM D6400 standard.

Preferably, the container compartment (4) comprises a first volume fraction and a second volume fraction, wherein the first volume fraction is occupied by the single-dose composition, preferably for about two-thirds of a total internal volume of said compartment (4), and wherein the second volume fraction is devoid of said composition, preferably for about one-third of the total internal volume.

Preferably, the single-dose composition of powder mannitol contained in said container (1), as above disclosed, is for use in the treatment of constipation, or for use as purgative to be administered to a patient prior to performing an endoscopic examination.

### LIST OF REFERENCE NUMBERS

| | | | |
|---|---|---|---|
| 1 | container, preferably single-dose container | 40 | first axial end |
| | | 42 | second axial end |
| 2 | first lateral wall | 44 | access opening |
| 4 | containment compartment | 46 | coupling seat or housing seat |
| 6 | sealing element | 48 | recess |
| 8 | first bottom wall | 50 | bellows-like portion |
| 10 | kit | 52 | outer tubular or annular portions |
| 12 | first axial end | 54 | inner tubular or annular portions |
| 14 | second axial end | 56 | material film |
| 16 | access opening | 58 | material film |
| 18 | first volume fraction | 60 | peripheral portions |
| 20 | second volume fraction | 62 | self-supporting bottom |
| 22 | vacant surface | 64 | powder-loading portion |
| 24 | funnel | 66 | powder-discharge portion |
| 26 | inner compartment | 68 | cap or closure member |
| 28 | level gauge | 70 | container neck |
| 30 | dissolving container | 72 | complementary threaded means |
| 32 | tamper-proof seal | X | first main extension axis |
| 34 | second lateral wall | Y | second main extension axis |
| 36 | window or portion permeable to light | | |
| 38 | second bottom wall | | |

## Claims

1. A single-dose composition comprising or consisting of mannitol in an amount comprised from 50 to 200 grams, wherein said mannitol is in form of a powder, wherein said powder has a bulk density comprised from 0.50 to 0.62 g/ml and it comprises powder particles, wherein a percentage comprised from 90% to 100% by weight of the powder particles has a particle size dimension comprised from 1 µm to 500 µm.

2. The single-dose composition according to the preceding claim, wherein the mannitol has a percentage by weight comprised from 97% to 100%, preferably a percentage by weight of 100%, with respect to the overall weight of said composition, wherein said bulk density of said powder is comprised from 0.50 g/ml to 0.62 g/ml, and wherein the single-dose composition devoid of excipients and/or pyrogenic substances.

3. The single-dose composition according to any one of the preceding claims, for use in the treatment of constipation, or for use as a purgative to be administered to a patient prior to a colonoscopy examination.

4. A container (1), preferably a single-dose container, containing a single-dose composition according to claim 1 or 2 in a container compartment (4) hermetically sealed by a removable sealing element (6).

5. A heat-sealed sachet, preferably a single-dose heat-sealed sachet, containing a single-dose composition according to claim 1 or 2 in a container compartment (4) hermetically sealed by a removable or tearable sealing element, wherein said heat-sealed sachet is made of a polymeric material compostable according to the UNI EN 13432 or ASTM D6400 standard.

6. The container according to claim 4 or the sachet according to claim 5, wherein the container compartment (4) comprises a first volume fraction and a second volume fraction, wherein the first volume fraction is occupied by the single-dose composition, preferably for about two-thirds of a total internal volume of said compartment (4), and wherein the second volume fraction is devoid of said composition, preferably for about one-third of the total internal volume.

7. A kit comprising:
- a container (1) according to claim 4 or 6 or the heat-sealed sachet according to claim 5 or 6;
- a dissolving container (30) at least partially permeable to light:
a) in which a volume of water suitable to fully dissolve the single-dose composition contained in the container (1) or in the heat-sealed sachet is contained;
or
b) comprising a level gauge (28) for a volume of water that can be introduced into the dissolving container (30) and suitable to fully dissolve the single-dose composition contained in the container (1) or in the heat-sealed sachet; and
- a funnel (24) for transferring the single-dose composition from the container (1) to the dissolving container (30).

8. The kit according to the preceding claim, wherein the ratio between the weight of the mannitol contained in the container (1) or in the heat-sealed sachet and the water volume contained or that can be introduced up to the level gauge (28) in the dissolving container (30) are mutually selected so as to obtain - at a temperature of 25°C - a concentration of mannitol in aqueous solution comprised from 0.05 g/ml to 0.213 g/ml, preferably comprised from 0.1 g/ml to 0.19 g/ml,

9. The kit according to any one of claims 7-8, wherein:
- the container (1) or the heat-sealed sachet is shape-coupled to the, or it is at least partially nested in the in the dissolving container (30); or
- at least one wall of the dissolving container (30) is flexible so that an internal compartment (26) of said container is expandable from an at least partially collapsed configuration to an expanded configuration.

10. A method for using the kit according to any one of claims 7-9, comprising the following steps:
i) removing the sealing element (6) from the container (1) or from the heat-sealed sachet;
ii) transferring the single-dose composition from the container (1) or from the heat-sealed sachet to the dissolving container (30) using the funnel (24);
iii) completely dissolving the mannitol powder in the water volume thus obtaining a single-dose solution.

11. The method according to the preceding claim, wherein:
- step ii) comprises the following sub-steps:
ii.a) first pouring at least one part of the appropriate water volume into the container (1) or into the heat-sealed sachet, so as to pre-dissolve the mannitol powder;
ii.b) subsequently transferring the mannitol pre-dissolution of sub-step ii.a) into the dissolving container (30); and/or
- wherein step iii) comprises the following sub-steps:
iii.a) hermetically sealing the dissolving container (30);
iii.b) shaking the dissolving container (30) of sub-step iii.a) until, using a portion (36) of said container at least partially permeable to light, the precipitates / sediments in the single-dose solution disappear.

12. A container (1), preferably a single-dose container or a single-dose heat-sealed sachet, containing a single-dose composition of mannitol in powder form as defined in claim 1; said mannitol in powder form being obtainable or obtained by means of a method comprising the following steps:
a) breaking-up a coherent mass of powder mannitol, so as to obtain a disintegrated mass from said coherent mass;
b) filling a plurality of containers with the broken-up mass of step a);
wherein step a) comprises a breaking-up with a centrifugal force, and wherein a bulk density of the coherent mass is smaller than the bulk density of the broken-up mass.

13. The container (1) according to claim 12, wherein said bulk density of the coherent mass is smaller than a bulk density of the broken-up mass by a percentage comprised from 1% to 40%, preferably comprised from 1% to 30%, even more preferably comprised from 5% to 15%, with respect to the bulk density of the broken-up mass.

14. The container (1) according to claim 12 or 13, wherein said centrifugal force is exerted by means of drum centrifugal sieve shaker.

15. The container (1) according to any one of claims 12-14, wherein step a) comprises the following sub-steps:
a.i) sieving the coherent mass of mannitol powder; and
a.ii) packing the product of sub-step a.i).

16. The container (1) according to any one of claims 12-15, wherein - in sub-step a.ii) - the powder mannitol is forced through a mesh with a clear span comprised from 2.0 to 5.0 millimetres, preferably comprised from 2.0 to 4.0 millimetres, more preferably comprised from 2.5 to 3.5 millimetres, even more preferably of 3.0 millimetres.

17. The container (1) according to any one of claims 12-16, wherein at the end of step b), the mannitol powder in the container has a bulk density comprised from 0.50 to 0.62 g/ml.

18. The container (1) according to any one of claims 12-17, wherein at the end of step b), the mannitol powder comprises an amount comprised from 90% to 100% by weight of powder particles with a particle size dimension comprised from 1 µm to 500 µm preferably comprised from 1 µm to 400 µm, more preferably comprised from 1 µm to 300 µm.

19. The container (1) according to any one of claims 12-18, wherein the single-dose composition is devoid of excipients and/or pyrogenic substances.

20. The container (1) according to any one of claims 12-19, wherein the container (1) delimits a container compartment (4), wherein the container compartment (4) comprises a first volume fraction and a second volume fraction, and wherein - at the end of step b) - the first volume fraction is occupied by the single-dose composition, and the second volume fraction of said compartment is devoid of said composition, and preferably wherein the first volume fraction is about two-thirds of a total internal volume of the container compartment (4), the second volume fraction being about one-third of the total internal volume.

21. The container (1) according to any one of claims 12-20, wherein said single-dose composition comprises, or consists, of mannitol at an amount comprised from 50 to 200 grams, wherein said mannitol is in form of powder, wherein said powder has a bulk density comprised frorr 0.50 to 0.62 g/ml and it comprises powder particles, and wherein a percentage comprised from 90% to 100% by weight of the powder particles has a particle size dimension comprised from 1 µm to 500 µm.

22. The container (1) according to any one of claims 12-21, wherein the mannitol contained in said single-dose composition is present at a percentage by weight comprised from 97% to 100%, preferably a percentage by weight of 100%, with respect to the total weight of said composition, wherein said bulk density of said mannitol powder is comprised from 0.50 g/ml to 0.62 g/ml, and wherein the single-dose composition is devoid of excipients and/or pyrogenic substances.

23. The container (1), preferably a single-dose container or a single-dose heat-sealed sachet, wherein said single-dose composition according to any one of claims 12-22, is contained in a container compartment (4) hermetically sealed by a removable sealing element (6).

24. The container (1) according to any one of claims 12-23, wherein said container is a heat-sealed sachet, preferably a single-dose heat-sealed sachet, containing a single-dose composition according to any one of claims 12-23, in a container compartment (4) hermetically sealed by a removable or tearable sealing element, wherein said heat-sealed sachet is made of a polymeric material compostable according to the UNI EN 13432 or ASTM D6400 standard.

25. The container (1) according to any one of claims 12-24, wherein the container compartment (4) comprises a first volume fraction and a second volume fraction, wherein the first volume fraction is occupied by the single-dose composition, preferably for about two-thirds of a total internal volume of said compartment (4), and wherein the second volume fraction is devoid of said composition, preferably for about one-third of the total internal volume.

## Patentansprüche

1. Einzeldosiszusammensetzung, umfassend oder bestehend aus Mannit in einer Menge im Bereich von 50 bis 200 Gramm, wobei das Mannit in Form eines Pulvers vorliegt, wobei das Pulver eine Schüttdichte im Bereich von 0,50 bis 0,62 g/ml aufweist und es Pulverteilchen umfasst, wobei ein Prozentsatz im Bereich von 90 % bis 100 Gew.- % der Pulverteilchen eine Teilchengrößenabmessung im Bereich von 1 µm bis 500 µm aufweist.

2. Einzeldosiszusammensetzung nach dem vorhergehenden Anspruch, wobei das Mannit einen Gewichtsprozentsatz im Bereich von 97 % bis 100 %, vorzugsweise einen Gewichtsprozentsatz von 100 %, bezogen auf das Gesamtgewicht der Zusammensetzung, aufweist, wobei die Schüttdichte des Pulvers im Bereich von 0,50 g/ml bis 0,62 g/ml liegt und wobei die Einzeldosiszusammensetzung frei von Hilfsstoffen und/oder pyrogenen Substanzen ist.

3. Einzeldosiszusammensetzung nach einem der vorhergehenden Ansprüche zur Verwendung bei der Behandlung von Verstopfung oder zur Verwendung als Abführmittel, die an eine(n) Patienten/in vor einer Koloskopieuntersuchung verabreicht werden soll.

4. Behälter (1), vorzugsweise Einzeldosisbehälter, enthaltend eine Einzeldosiszusammensetzung nach Anspruch 1 oder 2 in einer Behälterunterteilung (4), die durch ein entfernbares Versiegelungselement (6) hermetisch versiegelt ist.

5. Heißversiegelter Beutel, vorzugsweise heißversiegelter Einzeldosisbeutel, der eine Einzeldosiszusammensetzung nach Anspruch 1 oder 2 in einer Behälterunterteilung (4) enthält, die durch ein entfernbares oder aufreißbares Versiegelungselement hermetisch versiegelt ist, wobei der heißversiegelte Beutel aus einem polymeren Material hergestellt ist, das gemäß der Norm UNI EN 13432 oder ASTM D6400 kompostierbar ist.

6. Behälter nach Anspruch 4 oder Beutel nach Anspruch 5, wobei die Behälterunterteilung (4) eine erste Volumenfraktion und eine zweite Volumenfraktion umfasst, wobei die erste Volumenfraktion von der Einzeldosiszusammensetzung eingenommen wird, vorzugsweise für etwa zwei Drittel eines gesamten Innenvolumens der Unterteilung (4), und wobei die zweite Volumenfraktion frei von der Zusammensetzung ist, vorzugsweise für etwa ein Drittel des gesamten Innenvolumens.

7. Kit, umfassend:
- einen Behälter (1) nach Anspruch 4 oder 6 oder den heißversiegelten Beutel nach Anspruch 5 oder 6;
- einen Auflösungsbehälter (30), der zumindest teilweise lichtdurchlässig ist:
a) in dem ein Wasservolumen, das geeignet ist, die in dem Behälter (1) oder in dem heißversiegelten Beutel enthaltene Einzeldosiszusammensetzung vollständig aufzulösen, enthalten ist;
oder
b) umfassend eine Füllstandsanzeige (28) für ein Wasservolumen, das in den Auflösungsbehälter (30) eingeführt werden kann und geeignet ist, die in dem Behälter (1) oder in dem heißversiegelten Beutel enthaltene Einzeldosiszusammensetzung vollständig aufzulösen; und
- einen Trichter (24) zum Transferieren der Einzeldosiszusammensetzung aus dem Behälter (1) in den Auflösungsbehälter (30).

8. Kit nach dem vorhergehenden Anspruch, wobei das Verhältnis zwischen dem Gewicht des in dem Behälter (1) oder in dem heißversiegelten Beutel enthaltenen Mannits und dem Wasservolumen, das bis zu der Füllstandsanzeige (28) in dem Auflösungsbehälter (30) enthalten ist oder eingeführt werden kann, gegenseitig so gewählt ist, dass - bei einer Temperatur von 25 °C - eine Konzentration von Mannit in wässriger Lösung im Bereich von 0,05 g/ml bis 0,213 g/ml, vorzugsweise im Bereich von 0,1 g/ml bis 0,19 g/ml, erhalten wird.

9. Kit nach einem der Ansprüche 7-8, wobei:
- der Behälter (1) oder der heißversiegelte Beutel mit dem Auflösungsbehälter (30) formschlüssig gekoppelt ist oder zumindest teilweise darin verschachtelt ist; oder
- zumindest eine Wand des Auflösungsbehälters (30) flexibel ist, so dass eine innere Unterteilung (26) des Behälters von einer zumindest teilweise zusammengeklappten Auslegung in eine aufgeweitete Auslegung erweiterbar ist.

10. Verfahren zur Verwendung des Kits nach einem der Ansprüche 7-9, umfassend die folgenden Schritte:
i) Entfernen des Versiegelungselements (6) aus dem Behälter (1) oder aus dem heißversiegelten Beutel;
ii) Transferieren der Einzeldosiszusammensetzung aus dem Behälter (1) oder aus dem heißversiegelten Beutel in den Auflösungsbehälter (30) unter Verwendung des Trichters (24);
iii) vollständiges Auflösen des Mannitpulvers im Wasservolumen, wodurch eine Einzeldosislösung erhalten wird.

11. Verfahren nach dem vorhergehenden Anspruch, wobei:
- der Schritt ii) die folgenden Unterschritte umfasst:
ii.a) Gießen zuerst mindestens eines Teils des geeigneten Wasservolumens in den Behälter (1) oder in den heißversiegelten Beutel, um das Mannitpulver vorzulösen;
ii.b) anschließendes Transferieren der Mannit-Vorauflösung aus Unterschritt ii.a) in den Auflösungsbehälter (30);
und/oder
- wobei der Schritt iii) die folgenden Unterschritte umfasst:
iii.a) hermetisches Versiegeln des Auflösungsbehälters (30);
iii.b) Schütteln des Auflösungsbehälters (30) aus Unterschritt iii.a), bis, unter Verwendung eines Abschnitts (36) des Behälters, der zumindest teilweise lichtdurchlässig ist, die Niederschläge / Sedimente in der Einzeldosislösung verschwinden.

12. Behälter (1), vorzugsweise Einzeldosisbehälter oder heißversiegelter Einzeldosisbeutel, der eine Einzeldosiszusammensetzung von Mannit in Pulverform nach Anspruch 1 enthält; wobei das Mannit in Pulverform durch ein Verfahren erhältlich ist oder erhalten wird, das die folgenden Schritte umfasst:
a) Aufbrechen einer kohärenten Masse von Mannitpulver, um aus der kohärenten Masse eine zerbrochene Masse zu erhalten;
b) Füllen einer Vielzahl von Behältern mit der aufgebrochenen Masse aus Schritt a);
wobei der Schritt a) ein Aufbrechen mit einer Zentrifugalkraft umfasst, und wobei eine Schüttdichte der kohärenten Masse kleiner als die Schüttdichte der aufgebrochenen Masse ist.

13. Behälter (1) nach Anspruch 12, wobei die Schüttdichte der kohärenten Masse um einen Prozentsatz im Bereich von 1 % bis 40 %, vorzugsweise im Bereich von 1 % bis 30 %, noch bevorzugter im Bereich von 5 % bis 15 %, bezogen auf die Schüttdichte der aufgebrochenen Masse, kleiner als eine Schüttdichte der aufgebrochenen Masse ist.

14. Behälter (1) nach Anspruch 12 oder 13, wobei die Zentrifugalkraft mittels eines Trommelzentrifugalsiebschüttlers ausgeübt wird.

15. Behälter (1) nach einem der Ansprüche 12-14, wobei der Schritt a) die folgenden Unterschritte umfasst:
a.i) Sieben der kohärenten Masse des Mannitpulvers; und
a.ii) Verpacken des Produkts aus Unterschritt a.i).

16. Behälter (1) nach einem der Ansprüche 12-15, wobei - in Unterschritt a.ii) - das Mannitpulver durch ein Sieb mit einer freien Spannweite im Bereich von 2,0 bis 5,0 Millimeter, vorzugsweise im Bereich von 2,0 bis 4,0 Millimeter, bevorzugter im Bereich von 2,5 bis 3,5 Millimeter, noch bevorzugter von 3,0 Millimeter, gepresst wird.

17. Behälter (1) nach einem der Ansprüche 12-16, wobei am Ende von Schritt b) das Mannitpulver im Behälter eine Schüttdichte im Bereich von 0,50 bis 0,62 g/ml aufweist.

18. Behälter (1) nach einem der Ansprüche 12-17, wobei am Ende von Schritt b) das Mannitpulver eine Menge im Bereich von 90 % bis 100 Gew.- % Pulverteilchen mit einer Teilchengrößenabmessung im Bereich von 1 µm bis 500 µm, vorzugsweise im Bereich von 1 µm bis 400 µm, bevorzugter im Bereich von 1 µm bis 300 µm, umfasst.

19. Behälter (1) nach einem der Ansprüche 12-18, wobei die Einzeldosiszusammensetzung frei von Hilfsstoffen und/oder pyrogenen Substanzen ist.

20. Behälter (1) nach einem der Ansprüche 12-19, wobei der Behälter (1) eine Behälterunterteilung (4) begrenzt, wobei die Behälterunterteilung (4) eine erste Volumenfraktion und eine zweite Volumenfraktion umfasst, und wobei - am Ende von Schritt b) - die erste Volumenfraktion von der Einzeldosiszusammensetzung eingenommen wird und die zweite Volumenfraktion der Unterteilung frei von der Zusammensetzung ist, und wobei vorzugsweise die erste Volumenfraktion etwa zwei Drittel des gesamten Innenvolumens der Behälterunterteilung (4) beträgt, wobei die zweite Volumenfraktion etwa ein Drittel des gesamten Innenvolumens beträgt.

21. Behälter (1) nach einem der Ansprüche 12-20, wobei die Einzeldosiszusammensetzung Mannit in einer Menge im Bereich von 50 bis 200 Gramm umfasst oder daraus besteht, wobei das Mannit in Form eines Pulvers vorliegt, wobei das Pulver eine Schüttdichte im Bereich von 0,50 bis 0,62 g/ml aufweist und es Pulverteilchen umfasst, und wobei ein Prozentsatz im Bereich von 90 % bis 100 Gew.- % der Pulverteilchen eine Teilchengrößenabmessung im Bereich von 1 µm bis 500 µm aufweist.

22. Behälter (1) nach einem der Ansprüche 12-21, wobei das in der Einzeldosiszusammensetzung enthaltene Mannit in einem Gewichtsprozentsatz im Bereich von 97 % bis 100 %, vorzugsweise einem Gewichtsprozentsatz von 100 %, bezogen auf das Gesamtgewicht der Zusammensetzung, vorhanden ist, wobei die Schüttdichte des Mannitpulvers im Bereich von 0,50 g/ml bis 0,62 g/ml liegt und wobei die Einzeldosiszusammensetzung frei von Hilfsstoffen und/oder pyrogenen Substanzen ist.

23. Behälter (1), vorzugsweise Einzeldosisbehälter oder heißversiegelter Einzeldosisbeutel, wobei die Einzeldosiszusammensetzung nach einem der Ansprüche 12-22 in einer Behälterunterteilung (4) enthalten ist, die durch ein entfernbares Versiegelungselement (6) hermetisch versiegelt ist.

24. Behälter (1) nach einem der Ansprüche 12-23, wobei der Behälter ein heißversiegelter Beutel, vorzugsweise ein heißversiegelter Einzeldosisbeutel, ist, der eine Einzeldosiszusammensetzung nach einem der Ansprüche 12-23 in einer Behälterunterteilung (4) enthält, die durch ein entfernbares oder aufreißbares Versiegelungselement hermetisch versiegelt ist, wobei der heißversiegelte Beutel aus einem polymeren Material hergestellt ist, das gemäß der Norm UNI EN 13432 oder ASTM D6400 kompostierbar ist.

25. Behälter (1) nach einem der Ansprüche 12-24, wobei die Behälterunterteilung (4) eine erste Volumenfraktion und eine zweite Volumenfraktion umfasst, wobei die erste Volumenfraktion von der Einzeldosiszusammensetzung eingenommen wird, vorzugsweise für etwa zwei Drittel eines gesamten Innenvolumens der Unterteilung (4), und wobei die zweite Volumenfraktion frei von der Zusammensetzung ist, vorzugsweise für etwa ein Drittel des gesamten Innenvolumens.

## Revendications

1. Composition à dose unique, comprenant ou étant constituée de mannitol dans une quantité comprise entre 50 et 200 grammes, dans laquelle ledit mannitol se présente sous forme de poudre, dans laquelle ladite poudre a une masse volumique apparente comprise de 0,50 à 0,62 g/ml et comprend des particules de poudre, dans laquelle un pourcentage compris de 90 % à 100 % en poids des particules de poudre a une dimension de taille de particule comprise de 1 µm à 500 µm.

2. Composition à dose unique selon la revendication précédente, dans laquelle le mannitol a un pourcentage en poids compris de 97 % à 100 %, de préférence un pourcentage en poids de 100 %, par rapport au poids total de ladite composition, dans laquelle ladite masse volumique apparente de ladite poudre est comprise de 0,50 g/ml à 0,62 g/ml, et dans laquelle la composition à dose unique est dépourvue d'excipients et/ou de substances pyrogènes.

3. Composition à dose unique selon l'une quelconque des revendications précédentes, destinée à être utilisée dans le traitement de la constipation, ou à être utilisée comme purgatif à administrer à un patient avant un examen de coloscopie.

4. Récipient (1), de préférence récipient à dose unique, contenant une composition à dose unique selon la revendication 1 ou 2, dans un compartiment (4) de récipient hermétiquement fermé par un élément d'étanchéité (6) amovible.

5. Sachet thermoscellé, de préférence sachet thermoscellé à dose unique, contenant une composition à dose unique selon la revendication 1 ou 2, dans un compartiment (4) de récipient hermétiquement fermé par un élément d'étanchéité amovible ou déchirable, dans lequel ledit sachet thermoscellé est fabriqué à partir d'un matériau polymère compostable selon la norme UNI EN 13432 ou ASTM D6400.

6. Récipient selon la revendication 4 ou sachet selon la revendication 5, dans lequel le compartiment (4) de récipient comprend une première fraction volumique et une seconde fraction volumique, dans lequel la première fraction volumique est occupée par la composition à dose unique, de préférence pour environ deux tiers du volume interne total dudit compartiment (4), et dans lequel la seconde fraction volumique est dépourvue de ladite composition, de préférence pour environ un tiers du volume interne total.

7. Kit, comprenant :
- un récipient (1) selon la revendication 4 ou 6 ou le sachet thermoscellé selon la revendication 5 ou 6 ;
- un récipient de dissolution (30) au moins partiellement perméable à la lumière :
a) dans lequel est contenu un volume d'eau adapté pour dissoudre complètement la composition à dose unique contenue dans le récipient (1) ou dans le sachet thermoscellé ;
ou
b) comprenant un indicateur de niveau (28) pour un volume d'eau pouvant être introduit dans le récipient de dissolution (30) et adapté pour dissoudre complètement la composition à dose unique contenue dans le récipient (1) ou dans le sachet thermoscellé ; et
- un entonnoir (24) pour transférer la composition à dose unique du récipient (1) vers le récipient de dissolution (30).

8. Kit selon la revendication précédente, dans lequel le rapport entre le poids du mannitol contenu dans le récipient (1) ou dans le sachet thermoscellé et le volume d'eau contenu ou pouvant être introduit jusqu'à l'indicateur de niveau (28) dans le récipient de dissolution (30) est mutuellement choisi de manière à obtenir - à une température de 25 °C - une concentration de mannitol en solution aqueuse comprise de 0,05 g/ml à 0,213 g/ml, de préférence comprise de 0,1 g/ml à 0,19 g/ml.

9. Kit selon l'une quelconque des revendications 7-8, dans lequel :
- le récipient (1) ou le sachet thermoscellé est couplé par forme au récipient de dissolution (30) ou est au moins partiellement imbriqué dans celui-ci ; ou
- au moins une paroi du récipient de dissolution (30) est flexible de sorte qu'un compartiment interne (26) dudit récipient peut être expansible d'une configuration au moins partiellement repliée à une configuration déployée.

10. Procédé destiné à utiliser le kit selon l'une quelconque des revendications 7-9, comprenant les étapes suivantes :
i) retirer l'élément d'étanchéité (6) du récipient (1) ou du sachet thermoscellé ;
ii) transférer la composition à dose unique du récipient (1) ou du sachet thermoscellé vers le récipient de dissolution (30) à l'aide de l'entonnoir (24) ;
iii) dissoudre complètement la poudre de mannitol dans le volume d'eau pour obtenir ainsi une solution à dose unique.

11. Procédé selon la revendication précédente, dans lequel :
- l'étape ii) comprend les sous-étapes suivantes :
ii.a) verser d'abord au moins une partie du volume d'eau approprié dans le récipient (1) ou dans le sachet thermoscellé, de manière à pré-dissoudre la poudre de mannitol ;
ii.b) transférer ensuite la pré-dissolution de mannitol de la sous-étape ii.a) dans le récipient de dissolution (30) ;
et/ou
- dans lequel l'étape iii) comprend les sous-étapes suivantes :
iii.a) fermer hermétiquement le récipient de dissolution (30) ;
iii.b) agiter le récipient de dissolution (30) de la sous-étape iii.a) jusqu'à ce que, en utilisant une partie (36) dudit récipient au moins partiellement perméable à la lumière, les précipités / sédiments dans la solution à dose unique disparaissent.

12. Récipient (1), de préférence récipient à dose unique ou sachet thermoscellé à dose unique, contenant une composition à dose unique de mannitol sous forme de poudre telle que définie dans la revendication 1 ; ledit mannitol sous forme de poudre pouvant être obtenu ou étant obtenu au moyen d'un procédé comprenant les étapes suivantes :
a) désagréger une masse cohérente de poudre de mannitol, de manière à obtenir une masse désintégrée à partir de ladite masse cohérente ;
b) remplir une pluralité de récipients avec la masse désagrégée de l'étape a) ;
dans lequel l'étape a) comprend une désagrégation par force centrifuge, et dans lequel la masse volumique apparente de la masse cohérente est inférieure à la masse volumique apparente de la masse désagrégée.

13. Récipient (1) selon la revendication 12, dans lequel ladite masse volumique apparente de la masse cohérente est inférieure à une masse volumique apparente de la masse désagrégée selon un pourcentage compris de 1 % à 40 %, de préférence compris de 1 % à 30 %, encore plus préférablement compris de 5 % à 15 %, par rapport à la masse volumique apparente de la masse désagrégée.

14. Récipient (1) selon la revendication 12 ou 13, dans lequel ladite force centrifuge est exercée au moyen d'une machine à secouer les tamis centrifuge à tambour.

15. Récipient (1) selon l'une quelconque des revendications 12-14, dans lequel l'étape a) comprend les sous-étapes suivantes :
a.i) tamiser la masse cohérente de poudre de mannitol ; et
a.ii) conditionner le produit de la sous-étape a.i).

16. Récipient (1) selon l'une quelconque des revendications 12-15, dans lequel - dans la sous-étape a.ii) - la poudre de mannitol est forcée à passer à travers une structure à mailles dont la portée libre est comprise de 2,0 à 5,0 millimètres, de préférence de 2,0 à 4,0 millimètres, plus préférablement de 2,5 à 3,5 millimètres, et encore plus préférablement est de 3,0 millimètres.

17. Récipient (1) selon l'une quelconque des revendications 12-16, dans lequel, à la fin de l'étape b), la poudre de mannitol dans le récipient a une masse volumique apparente comprise entre 0,50 et 0,62 g/ml.

18. Récipient (1) selon l'une quelconque des revendications 12-17, dans lequel, à la fin de l'étape b), la poudre de mannitol comprend une quantité comprise de 90 % à 100 % en poids de particules de poudre ayant une dimension de taille de particule comprise de 1 µm à 500 µm, de préférence comprise de 1 µm à 400 µm, de préférence comprise de 1 µm à 300 µm.

19. Récipient (1) selon l'une quelconque des revendications 12-18, dans lequel la composition à dose unique est dépourvue d'excipients et/ou de substances pyrogènes.

20. Récipient (1) selon l'une quelconque des revendications 12-19, dans lequel le récipient (1) délimite un compartiment (4) de récipient, dans lequel le compartiment (4) de récipient comprend une première fraction volumique et une seconde fraction volumique, et dans lequel - à la fin de l'étape b) - la première fraction volumique est occupée par la composition à dose unique, et la seconde fraction volumique dudit compartiment est dépourvue de ladite composition, et de préférence dans lequel la première fraction volumique représente environ les deux tiers du volume interne total du compartiment (4) de récipient, la seconde fraction volumique représentant environ un tiers du volume interne total.

21. Récipient (1) selon l'une quelconque des revendications 12-20, dans lequel ladite composition à dose unique comprend ou est constituée de mannitol dans une quantité comprise de 50 à 200 grammes, dans lequel ledit mannitol se présente sous forme de poudre, dans lequel ladite poudre a une masse volumique apparente comprise de 0,50 à 0,62 g/ml et comprend des particules de poudre, et dans lequel un pourcentage compris de 90 % à 100 % en poids des particules de poudre a une dimension de taille de particule comprise de 1 µm à 500 µm.

22. Récipient (1) selon l'une quelconque des revendications 12-21, dans lequel le mannitol contenu dans ladite composition à dose unique est présent à un pourcentage en poids compris de 97 % à 100 %, de préférence un pourcentage en poids de 100 %, par rapport au poids total de ladite composition, dans lequel ladite masse volumique apparente de ladite poudre de mannitol est comprise de 0,50 g/ml à 0,62 g/ml, et dans lequel la composition à dose unique est dépourvue d'excipients et/ou de substances pyrogènes.

23. Récipient (1), de préférence récipient à dose unique ou sachet thermoscellé à dose unique, dans lequel ladite composition à dose unique selon l'une quelconque des revendications 12-22, est contenue dans un compartiment (4) de récipient hermétiquement fermé par un élément d'étanchéité (6) amovible.

24. Récipient (1) selon l'une quelconque des revendications 12-23, dans lequel ledit récipient est un sachet thermoscellé, de préférence un sachet thermoscellé à dose unique, contenant une composition à dose unique selon l'une quelconque des revendications 12-23, dans un compartiment (4) de récipient hermétiquement fermé par un élément d'étanchéité amovible ou déchirable, dans lequel ledit sachet thermoscellé est fabriqué à partir d'un matériau polymère compostable selon la norme UNI EN 13432 ou ASTM D6400.

25. Récipient (1) selon l'une quelconque des revendications 12-24, dans lequel le compartiment (4) de récipient comprend une première fraction volumique et une seconde fraction volumique, dans lequel la première fraction volumique est occupée par la composition à dose unique, de préférence pour environ deux tiers du volume interne total dudit compartiment (4), et dans lequel la seconde fraction volumique est dépourvue de ladite composition, de préférence pour environ un tiers du volume interne total.
